(19) Europäisches Patentamt
European Patent Office
Office européen des brevets

(11) EP 0 997 136 A1

(12) DEMANDE DE BREVET EUROPEEN

(43) Date de publication:
**03.05.2000 Bulletin 2000/18**

(51) Int Cl.[7]: **A61K 7/42**

(21) Numéro de dépôt: **99401882.8**

(22) Date de dépôt: **23.07.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **24.09.1998 FR 9811946**

(71) Demandeur: **L'OREAL**
**75008 Paris (FR)**

(72) Inventeurs:
- **Arnaud, Pascal**
**94240 L'Hay les Roses (FR)**
- **Viard, Martine**
**75013 Paris (FR)**

(74) Mandataire: **Miszputen, Laurent**
**L'OREAL-DPI**
**6 rue Bertrand Sincholle**
**92585 Clichy Cédex (FR)**

# (54) Compositions cosmétiques comprenant un dérivé silicié à fonction benzotriazole et un triester de triacide benzoique

(57) L'invention concerne de nouvelles compositions cosmétiques à usage topique, en particulier pour la photoprotection de la peau et/ou des cheveux, présentant un pouvoir photoprotecteur amélioré, et qui sont caractérisées par le fait qu'elles comprennent, dans un support cosmétiquement acceptable :

(i) au moins un dérivé silicié à fonction benzotriazole à titre de filtre organique solaire lipophile et ;
(ii) au moins un triester du triacide benzoïque en une quantité suffisante pour solubiliser à lui seul la totalité dudit filtre.

Application à la protection de la peau, des lèvres, des cheveux, des cils, des sourcils, des ongles contre les effets du rayonnement ultraviolet.

EP 0 997 136 A1

## Description

La présente invention concerne de nouvelles compositions cosmétiques à usage topique notamment destinées à la photoprotection de la peau et/ou des lèvres et/ou et/ou des phanères contre le rayonnement ultraviolet (compositions ci-après dénommées plus simplement compositions antisolaires), ainsi que leur utilisation dans de nombreuses applications cosmétiques. Plus précisément encore, elle concerne des compositions à pouvoir photoprotecteur amélioré, comprenant, dans un support cosmétiquement acceptable: (i) au moins un dérivé silicié à fonction benzotriazole à titre de filtre organique solaire lipophile et (ii) une huile particulière convenablement sélectionnée, présente en une quantité déterminée, et qui est un triester du triacide benzoïque.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires contiennent, à des concentrations diverses et selon la nature de la forme galénique choisie, un ou plusieurs filtres organiques classiques lipophiles et/ou hydrophiles capables d'absorber sélectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Il s'avère que des filtres organiques UV lipophiles particulièrement intéressants en cosmétique antisolaire et fortement actifs à la fois dans l'UV-A et dans l'UV-B ont été décrits dans les demandes EP-A-0392883; EP-A-0660701; EP-A-0708108 ; EP-A-0711778 ; EP-A-711779.

Il s'agit de silanes ou de polyorganosiloxanes à fonction benzotriazole. Ils présentent la particularité mais aussi le désavantage d'être solides à température ambiante. De ce fait, leur utilisation dans une composition cosmétique antisolaire implique certaines contraintes au niveau de leur formulation et de leur mise en oeuvre, en particulier lorsqu'il s'agit de trouver des solvants permettant de les solubiliser correctement. A cet égard, on fait aujourd'hui le plus souvent appel à des huiles comme des esters et plus particulièrement des benzoates d'alkyles en $C_{12}$-$C_{15}$ ("FINSOLV TN" de chez Finetex), ou à des triglycérides et notamment des triglycérides d'acides gras en $C_8$-$C_{12}$ ("MIGLYOL 812" de chez Hüls), ou encore à des monoalcools ou des polyols tels que l'éthanol, ainsi qu'à leurs mélanges. Ces produits, bien que possédant des propriétés solubilisantes vis à vis des filtres susmentionnés, présentent néanmoins pour inconvénient de n'avoir aucune activité propre dans le domaine de la filtration du rayonnement UV, tant UV-A qu'UV-B.

La présente invention vise à résoudre les problèmes ci-dessus.

L'utilisation des huiles du type triester du triacide benzoïque telles que le trimellitate de tridécyle est bien connue pour la formulation de nombreux produits cosmétiques comportant une phase grasse. Elle est décrite notamment dans la demande EP-A-0792637 relative à des rouges à lèvres. Elle est mentionnée également dans la demande EP-A-0194055 pour la préparation de formulations cosmétiques anhydres sans huile minérale.

Dans le brevet US4,940,577, ce type d'huile constitue la phase grasse de microémulsions transparentes du type eau-dans-huile à faible teneur en eau et contenant nécessairement comme unique émulsionnant un ester phosphate spécifique. Ces microémulsions sont utilisées notamment comme produits antisolaires à base de filtres UV organiques tels que le para-aminobenzoate d'octyldiméthyle, le cinnamate d'octyle, le salicylate d'octyle ou le benzophénone-3 ainsi que des produits autobronzants.

Dans le brevet US4,940,574, ce type d'huile est également utilisée comme émollient dans des produits solaires anhydres à haut degré de protection contenant dans une huile de silicone l'association de deux filtres organiques UV-B choisis parmi un cinnamate, un salicylate et un para-aminobenzoate et d'un filtre organique UV-A du type benzophénone.

A la suite d'importantes recherches menées dans le domaine de la photoprotection évoqué ci-dessus, la Demanderesse a maintenant découvert, de façon inattendue et surprenante, qu'il était possible d'améliorer de manière substantielle le pouvoir photoprotecteur d'un filtre UV lipophile du type dérivé silicié à fonction benzotriazole contenu dans les compositions cosmétiques photoprotectrices en associant ce filtre particulier avec une huile spécifique convenablement sélectionnée et qui est un triester du triacide benzoïque, cette huile devant être présente dans lesdites compositions en une quantité suffisante pour solubiliser à elle seule l'ensemble dudit filtre.

Cette découverte est à la base de la présente invention.

Ainsi, conformément à l'un des objets de la présente invention, il est maintenant proposé de nouvelles compositions cosmétiques ou dermatologiques, qui sont essentiellement caractérisées par le fait qu'elles comprennent, dans un support cosmétiquement acceptable, (i) au moins un filtre UV lipophile du type dérivé silicié à fonction benzotriazole, et (ii) au moins un triester du triacide benzoïque en une quantité suffisante pour solubiliser à lui seul la totalité dudit filtre.

La présente invention a également pour objet l'utilisation de telles compositions pour la fabrication de compositions destinées à la protection de la peau et/ou des lèvres et/ou des phanères telles que les cheveux, les cils, les sourcils ou les ongles contre le rayonnement ultraviolet, en particulier le rayonnement solaire.

Un autre objet enfin de la présente invention réside dans l'utilisation d'au moins un triester du triacide benzoïque dans la préparation d'une composition cosmétique de protection de la peau et/ou des lèvres et/ou des phanères telles que les cheveux, les cils, les sourcils ou les ongles contre le rayonnement ultraviolet, comprenant un système filtrant les radiations UV constitué d'au moins un dérivé silicié à fonction benzotriazole, en vue d'améliorer le pouvoir photoprotecteur de ladite composition.

D'autres caractéristiques, aspects et avantages de la présente invention apparaîtront à la lecture de la description détaillée qui va suivre.

Les dérivés siliciés à fonction benzotriazole utilisés dans la présente invention sont de préférence des silanes ou des siloxanes à fonction benzotriazole comprenant au moins une unité de formule (1) suivante :

$$O_{(3-a)/2}\ Si\ (R_1)_a\ -\ G \qquad (1)$$

dans laquelle :

- $R_1$ représente un radical alkyle en $C_1$-$C_{10}$ éventuellement halogéné ou un radical phényle ou un radical triméthylsilyloxy,
- a est un nombre entier choisi entre 0 et 3 inclusivement,
  - et le symbole G désigne un radical monovalent lié directement à un atome de silicium, et qui répond à la formule (2) suivante :

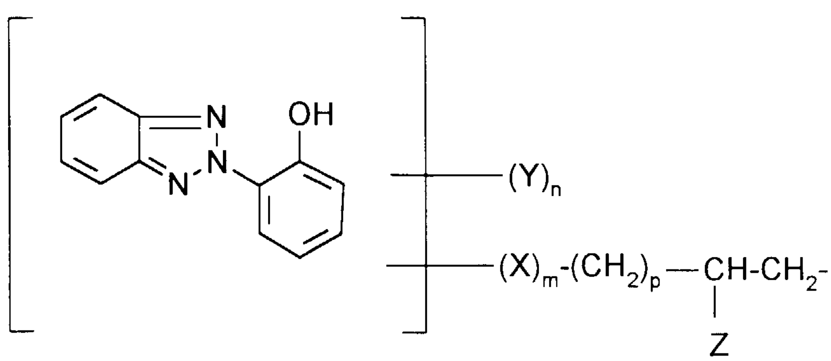

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,

- m est 0 ou 1,
- p représente un nombre entier compris entre 1 et 10, inclusivement.

Ces composés sont notamment décrits dans les demandes de brevet EP-A-0392883 ; EP-A-0660701 ; EP-A-0708108 ; EP-A-0711778 ; EP-A-711779.
De préférence, les dérivés siliciés utilisés dans le cadre de la présente invention appartiennent à la famille générale des silicones benzotriazoles qui est décrite notamment dans EP-A-0660701 .
Une famille de silicones benzotriazoles convenant particulièrement bien à la réalisation de la présente invention est celle regroupant les composés répondant aux formules (3) ou (4) suivantes :

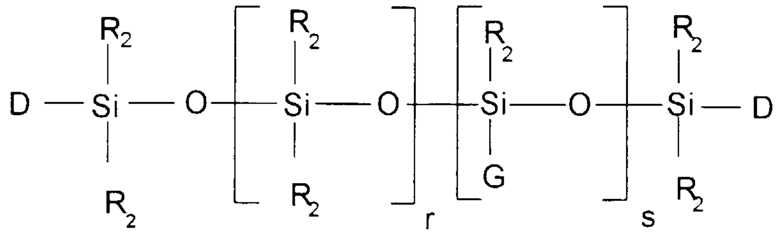

(3)

ou

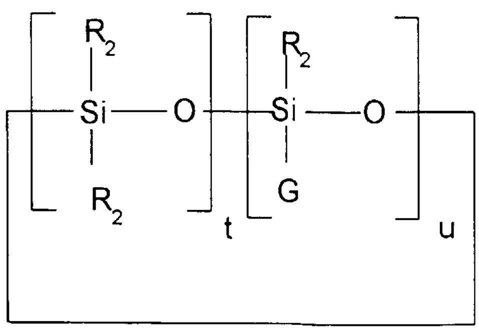

(4)

dans lesquelles :

- les radicaux $R_2$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_2$ étant méthyle,
- D, identiques ou différents sont choisis parmi les radicaux $R_2$ et le radical G,
- r est un nombre entier compris entre 0 et 50 inclusivement. et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) ci-dessus.

Comme cela ressort de la formule (2) donnée ci-dessus, l'accrochage du chaînon $-(X)_m-(CH_2)_p-CH(Z)-CH_2-$ sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

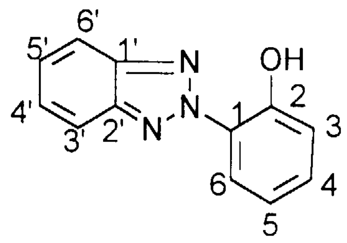

De préference, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (3) et (4) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle $R_2$ préférés selon l'invention sont les radicaux méthyle, éthyle, propyle, n-butyle, n-octyle et éthyl-2 hexyle. Encore plus préférentiellement, les radicaux $R_2$ sont tous des radicaux méthyle.

Parmi les composés de formules (3) ou (4) ci-dessus, on préfère mettre en oeuvre ceux répondant à la formule (3), c'est-à-dire des diorganosiloxanes à chaîne courte linéaire.

Parmi les composés de formules (3) ci-dessus, on préfère mettre en oeuvre ceux pour lesquels les radicaux D sont tous les deux des radicaux $R_2$

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :

- D est un radical $R_2$
- $R_2$ est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, tert.-butyle ou alcoxy en $C_1$-$C_4$,
- Z est hydrogène ou méthyle,
- m=0, ou [m=1 et X=O]
- p est égal à 1.

Une famille de silicones benzotriazoles convenant particulièrement bien à l'invention est celle définie par la formule générale (5) suivante :

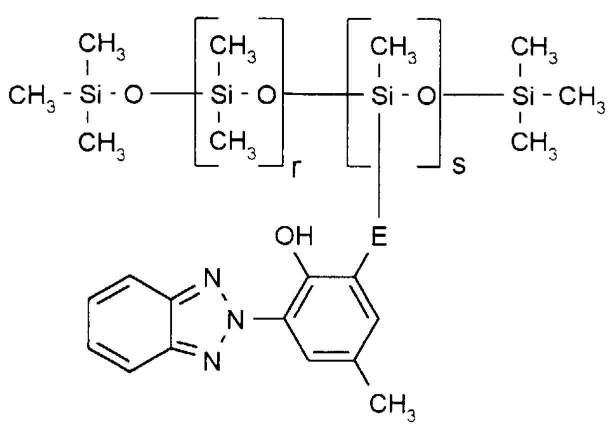

(5)

avec

$$0 \leq r \leq 10,$$

$$1 \leq s \leq 10,$$

et où E représente le radical divalent :

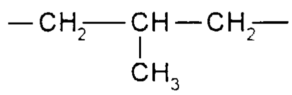

Dans une forme particulièrement préférée de réalisation de l'invention, la silicone benzotriazole est le composé (appelé composé (a) dans la suite du texte) répondant à la formule suivante :

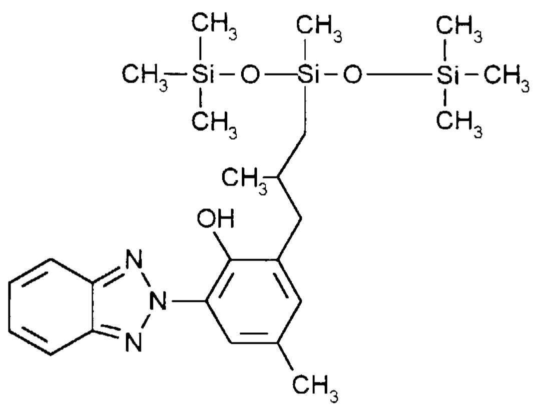

composé (a)

Des procédés convenant à la préparation des produits de formule (1), (3), (4) et (5) ci-dessus sont notamment décrits dans les brevets américains US 3, 220, 972, US 3, 697, 473, US 4, 340, 709, US 4, 316, 033, US 4, 328, 346 et dans les demandes de brevet EP-A-0 392 883 et EP-A-0 742 003.

Les filtres dérivés siliciés à fonction benzotriazole peuvent être présents dans les compositions selon l'invention à des teneurs allant de 0,1 à 20%, de préférence allant de 0,2 à15%, en poids, toujours par rapport au poids total de la composition. Selon une caractéristique essentielle de la présente invention, ces composés, pris seuls ou en mélanges, doivent se présenter dans la composition finale sous une forme totalement, ou substantiellement totalement, solubilisée.

Les triesters du triacide benzoïque utilisés conformément à la présente invention sont en général choisis parmi les triesters du triacide benzoïque avec des alcools linéaires ou ramifiés, saturés ou insaturés, ayant de 3 à 30 atomes de carbone et de préférence de 8 à 18 atomes de carbone. Ils répondent à la formule générale (I) suivante :

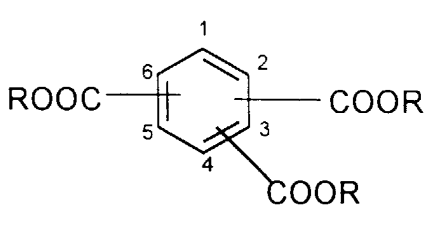

(I)

dans laquelle R désigne un alkyle linéaire ou ramifié, saturé ou insaturé, ayant de 3 à 30 atomes de carbone et de préférence de 8 à 18 atomes de carbone.

Ils sont choisis plus préférentiellement parmi les esters de l'acide trimellitique répondant à la formule générale (II) suivante :

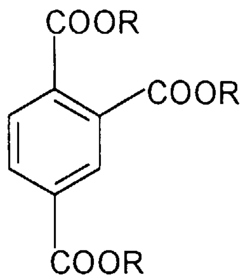

(II)

dans laquelle R a la même signification indiquée dans la formule (I) et plus particulièrement parmi :

- le trimellitate d'éthyl-2 hexyle tel que le produit commercial vendu sous le nom BISOFLEX TOT par la Société International Speciality Chemical ;
- le trimellitate de décyle ;
- le trimellitate de triisodécyle tel que le produit commercial vendu sous le nom DUB TMI par la Société STEARINERIES DUBOIS ;
- le trimellitate de butyl-2 hexyle tel que le produit commercial vendu sous le nom D'ISOFOL ESTER 1293 par la Société CONDEA;
- le trimellitate de tridécyle tel que le produit commercial vendu sous le nom LIPONATE TDTM par la Société LIPO CHEMICALS ;
- ou leurs mélanges.

Cette huile spécifique utilisée conformément à la présente invention est généralement présente dans les compositions finales à des teneurs comprises allant de 0,1 à 99% en poids par rapport au poids total de ladite composition, et de préférence à des teneurs allant de 0,5 à 50 % en poids.

Selon une caractéristique essentielle des compositions selon l'invention, le triester du triacide benzoïque doit être utilisé en une quantité telle qu'elle soit suffisante pour solubiliser à elle seule la totalité. ou substantiellement la totalité, du filtre silane. ou silicone à fonction benzotriazole présente dans la composition. Cette quantité minimale en huile solvante destinée à assurer une dissolution complète et stable du filtre solide peut être classiquement déterminée à partir d'essais de solubilité dudit filtre dans ce solvant.

D'une manière générale, on notera que les concentrations en filtre et en huile sont choisies de manière telle que l'indice de protection solaire de la composition finale soit de préférence d'au moins 2.

Les applications de l'association triester du triacide benzoïque/filtre UV du type dérivé silicié à fonction benzotriazole selon l'invention sont multiples et concernent l'ensemble des produits cosmétiques et dermatologiques.

Les produits cosmétiques ou dermatologiques de l'invention peuvent se présenter sous la forme de compositions solides, pâteuses ou liquides, anhydres ou en émulsion.

Ainsi, la composition de l'invention peut se présenter sous toutes les formes galéniques normalement utilisées pour une application topique, et notamment sous forme d'une huile ; d'une suspension ; d'un produit bi-phasé, d'une dispersion huile-dans-eau ou eau-dans-huile ; d'une émulsion simple ou complexe (huile-dans-eau , eau-dans-huile, eau-dans-huile-dans-eau ou huile-dans-eau-dans-huile) telle qu'une crème, un lait, un gel-crème, une pommade, un onguent; d'une dispersion vésiculaire ; d'une poudre ; d'un solide coulé ou moulé tel qu'un stick ou un produit compacté ; d'une mousse ou d'un spray.

Les compositions selon l'invention peuvent être avantageusement utilisées pour le maquillage et/ou le soin de la peau aussi bien du visage que du corps humain, des lèvres et des phanères comme les cheveux, les cils, les sourcils, les ongles selon la nature des actifs utilisés.

En particulier, les compositions de l'invention peuvent être un bâton de rouge à lèvres, un brillant à lèvres (gloss en terminologie anglo-saxonne) utilisable tel quel ou pour appliquer sur un film de rouge à lèvres notamment pour en augmenter sa brillance (top coat en terminologie anglo-saxonne). Elles peuvent aussi constituer un fond de teint fluide ou solide, un produit anti-cernes ou contours des yeux, un eye liner, un mascara, un fard à joues ou à paupières, un vernis à ongles, une poudre libre, un produit de maquillage du corps ou encore un produit de soin ou de nettoyage de la peau tel que les produits gommant. Ces compositions peuvent, en outre, contenir des actifs cosmétiques ou dermatologiques, en vue, notamment d'apporter un aspect soin ou traitant à la composition.

De façon plus spécifique, l'invention a pour objet un produit à lèvres contenant au moins l'association telle que définie précédemment.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants et notamment les antioxydants ARL, les opacifiants, les stabilisants, les émollients, les silicones, les composés fluorés, les α-hydroxyacides, les agents anti-mousse, les agents hydratants, les vitamines, les parfums, les conservateurs, les tensioactifs, les pigments, les nacres, les charges, les séquestrants. les polymères, les propulseurs, les agents alcalinisants ou acidifiants, les colorants, ou tout autre ingrédient habituellement utilisé en cosmétique

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium.

Parmi les nacres utilisables dans l'invention, on peut citer le mica recouvert d'oxyde de titane, d'oxyde de fer, de pigment naturel ou d'oxychlorure de bismuth tel que le mica titane coloré.

On peut notamment citer le talc, le mica, le kaolin, les poudres de Nylon (Orgasol notamment) et de polyéthylène, le Téflon, l'amidon, le nitrure de bore, des micro-sphères de copolymères telles que l'Expancel (Nobel Industrie), le polytrap (Dow Corning) et les microbilles de résine de silicone (Tospearl de Toshiba, par exemple).

Les corps gras pouvant être utilisés dans les compositions de l'invention sont généralement choisis en fonction de l'application envisagée parmi les huiles, les cires et les gommes ou leurs mélanges.

Les huiles peuvent être des huiles hydrocarbonées et/ou siliconées et/ou fluorées. Ces huiles peuvent être d'origine animale, végétale, minérale ou synthétique. A titre d'exemple d'huile utilisable dans l'invention, on peut citer les huiles hydrocarbonées d'origine animale telles que le perhydrosqualène ; les huiles hydrocarbonées végétales ou synthétiques telles que les triglycérides d'acides gras de 4 à 22 atomes de carbone comme les triglycérides des acides heptanoïque ou octanoïque, les triglycérides de noix de coco hydrogénés, et les triglycérides des acides caprylique/caprique comme ceux vendus par la société Stearineries Dubois ou ceux vendus sous les dénominations Miglyol 810, 812 et 818 par la société Dynamit; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme les huiles de formule $R_3COOR_4$ dans laquelle $R_3$ représente le reste d'un acide gras supérieur comportant de 7 à 29 atomes de carbone et $R_4$ représente une chaîne hydrocarbonée contenant de 3 à 30 atomes de carbone comme par exemple l'huile de Purcellin, le myristate d'isopropyle, le palmitate d'éthyl-2 hexyle, le stéarate d'octyl-2 dodécyle, l'érucate d'octyl-2 dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles siliconées comme les polyméthylsiloxanes (PDMS) volatiles ou non, linéaires ou cycliques, liquides ou pâteux à température ambiante ; leurs mélanges.

Les cires peuvent être hydrocarbonées, siliconées et/ou fluorées et être d'origine végétale, minérale, animale et/ou synthétique. En particulier, elles présentent une température de fusion supérieure à 25°C et mieux supérieure à 45°C.

Comme cire utilisable dans l'invention, on peut citer, la lanoline oxypropylénée ou non, acétylée ou non, la cire d'abeilles, la cire de Carnauba ou de Candellila, la paraffine, les cires de lignite ou microcristalline, la cérésine, ou l'ozokérite ; les cires synthétiques comme les cires de polyéthylène et les cires de Fischer-Tropsch ou encore l'octacosanylstéarate, les cires de silicones comme les alkyl ou alkoxydiméthicone ayant de 16 à 45 atomes de carbone.

La nature et la quantité des gommes ou cires sont fonction des propriétés mécaniques et des textures recherchées. A titre indicatif, la composition peut contenir de 0 à 50 % en poids de cires, par rapport au poids total de la composition et mieux de 5 à 30 %.

Les compositions cosmétiques selon l'invention peuvent être utilisées comme produits antisolaires pour la protection de la peau et/ou des cheveux contre les effets nocifs des radiations UV. Ces derniers peuvent se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâton solide tel qu'un stick, de mousse aérosol ou de spray.

Les compositions conformes à l'invention peuvent contenir un ou plusieurs filtres solaires complémentaires actifs dans l'UVA et/ou l'UVB (absorbeurs), hydrophiles ou lipophiles, autres bien sûr que le filtre mentionné ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres

décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions cosmétiques selon l'invention peuvent être également utilisées pour la protection des cheveux et peuvent se présenter sous forme de shampooing, de lotion, de gel, d'émulsion, de dispersion vésiculaire non ionique, de composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

**EXEMPLES 1 A 6:**

On a préparé diverses formulations antisolaires se présentant sous la forme d'une solution anhydre contenant :

- Silicone benzotriazole correspondant au composé (a) 5% en poids
- Huile 95% en poids

On fait varier la nature de l'huile utilisée.

On mesure pour chacune des solutions le spectre d'absorption UV entre 280 et 400 nm sur un spectrophotomètre UV Visible : MODELE 552 de PERKIN-ELMER muni d'un double faisceau et d'une lampe UV (Deutérium) et d'une lampe visible (Bromure de Tungstène). L'appareil est équipé d'une sphère d'intégration interne de chez PERKIN-ELMER.

Chacune des solutions non-diluées est étalée entre deux lames de quartz dont l'une est creuse de manière à obtenir une épaisseur de 10 µm.

On détermine sur chacun des spectres obtenus la densité optique aux longueurs d'onde correspondant au maximum d'absorption qui sont spécifiques au filtre siliconé à fonction benzotriazole utilisé à savoir : $\lambda_{1max}$ = 305 et $\lambda_{2max}$ = 347 nm

Les résultats sont indiqués dans le tableau A suivant :

Tableau A

| **Exemples** | **Huile utilisée** | **Densité optique à 305 nm** | **Densité optique à 347nm** |
|---|---|---|---|
| **1** (invention) | **Trimellitate de tridécyle**<br>(LIPONATE TDTM vendu par LIPO CHEMICALS) | **2,19** | **1,82** |
| **2** (art antérieur) | Phényltriméthicone<br>(DC 556 vendu par DOW CORNING) | 1,77 | 1,67 |
| **3** (art antérieur) | Triheptanoate de glycéryle<br>(LANOL 37T vendu par SEPPIC) | 1,61 | 1,55 |

Tableau A (suite)

| Exemples | Huile utilisée | Densité optique à 305 nm | Densité optique à 347nm |
|---|---|---|---|
| **4** (art antérieur) | Benzoate d'alkyle en C12-C15 (vendu par STEARINERIES DUBOIS) | 1,60 | 1,54 |
| **5** (art antérieur) | Néopentanoate d'octyldodécyle (ELEFAC 1 205 vendu par BERNEL) | 1,54 | 1,47 |
| **6** (art antérieur) | Octyldodécanol (EUTANOL G vendu par HENKEL) | 1,5 | 1,41 |

Ces résultats démontrent clairement par rapport aux huiles de l'art antérieur, que la présence du trimellitate de tridécyle augmente substantiellement le pouvoir d'absorption des radiations UV de la silicone benzotriazole utilisé comme filtre UV et dissoute dans ladite huile.

**EXEMPLES 7 à 10** : **Sticks de soin des lèvres**

On prépare 3 formulations sous forme de stick de soin pour les lèvres dont la composition est la suivante :

- Silicone benzotriazole correspondant au composé (a) 5% en poids
- Stéarate d'octacosanyle (Cire) vendu sous le nom KESTER WAX 82 H par la société KOSTER KEUNEN 10% en poids
- Huile 85% en poids

On fait varier la nature de l'huile utilisée.
Pour chacune des formulations ainsi préparées, on a ensuite déterminé le facteur de protection solaire (SPF) qui leur était attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.
Pour chacun des sticks, les mesures sont réalisées à l'aide d'un spectrophotomètre UV visible - modèle SPF 290 S de chez OPTOMETRICS - muni d'une lampe UV (Xénon) et d'une sphère d'intégration.
Chacune des composition est appliquée, sous forme de dépôt homogène à raison de $2mg/cm^2$, sur un ruban adhésif TRANSPORE de chez 3M collé sur lame de quartz.
Les compositions des différentes formulations étudiées et les résultats en facteur de protection moyen obtenus sont rassemblés dans le tableau B donné ci-dessous.

Tableau B

| Exemples | Huile utilisée | SPF moyen (écart type) |
|---|---|---|
| **7** (invention) | **Trimellitate de tridécyle** (LIPONATE TDTM vendu par LIPO CHEMICALS) | **6,3** **(0,2)** |
| **8** (art antérieur) | Triheptanoate de glycéryle (LANOL 37T vendu par SEPPIC) | 5,8 (0,3) |
| **9** (art antérieur) | Benzoate d'alkyle en $C_{12}$-$C_{15}$ (vendu par STEARINERIES DUBOIS) | 4,6 (0,5) |

Ces résultats démontrent clairement par rapport aux huiles de l'art antérieur. l'effet bénéfique remarquable apporté par la présence du trimellitate de tridécyle conforme à l'invention au niveau des facteurs de protection solaire des compositions finales.

## Revendications

**1.** Composition cosmétique ou dermatologique, caractérisée par le fait qu'elle comprend, dans un support cosmétiquement acceptable :

(i) au moins un dérivé silicié à fonction benzotriazole à titre de filtre organique solaire lipophile et ;
(ii) au moins un triester du triacide benzoïque en une quantité suffisante pour solubiliser à lui seul la totalité dudit filtre.

**2.** Composition selon la revendication 1, où le dérivé silicié à fonction benzotrazole est choisi parmi les silanes et/ou les polyorganosiloxanes à fonction benzotriazole comprenant au moins une unité de formule (1) suivante :

$$O_{(3-a)/2}\ Si\ (R_1)_a\text{- }G \qquad (1)$$

dans laquelle :

- $R_1$ représente un radical alkyle en $C_1$-$C_{10}$ éventuellement halogéné ou un radical phényle ou un radical triméthylsilyloxy,
- a est un nombre entier choisi entre 0 et 3 inclusivement,
  - et le symbole G désigne un radical monovalent lié directement à un atome de silicium, et qui répond à la formule (2) suivante :

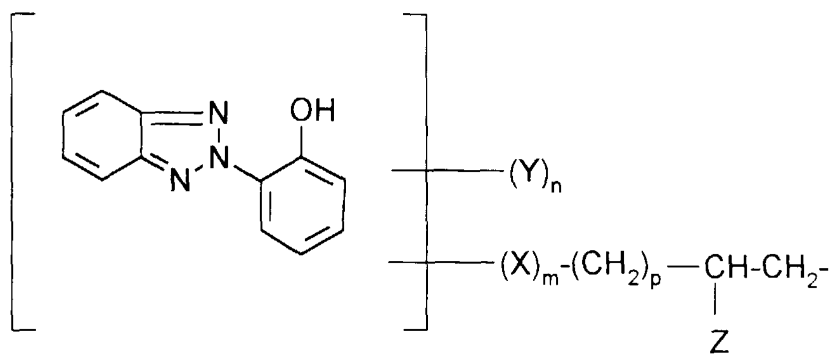

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

**3.** Composition selon la revendication 1 ou 2, caractérisée par le fait que le dérivé silicié à fonction benzotriazole répond à l'une des formules (3) ou (4) suivantes :

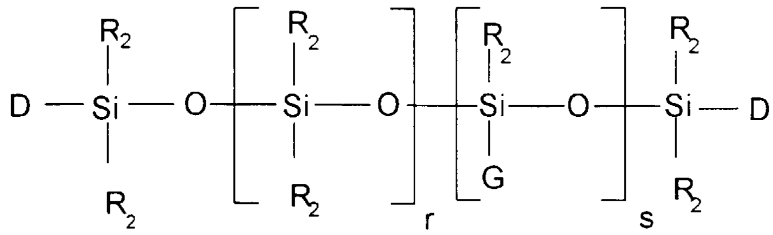 (3)

ou

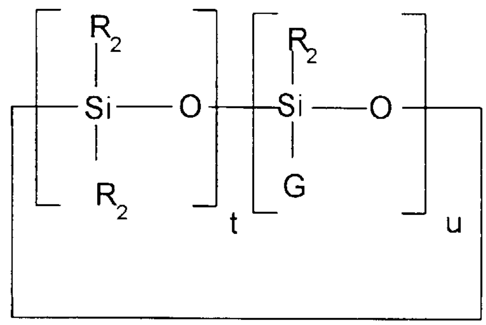 (4)

dans lesquelles :

- les radicaux $R_2$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_2$ étant méthyle,
- D, identiques ou différents sont choisis parmi les radicaux $R_2$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,

- et le symbole G répond à la formule (2) définie dans la revendication 2.9.

**4.** Composition selon la revendication 3, caractérisée par le fait que dérivé silicié à fonction benzotriazole répond à la formule (5) suivante :

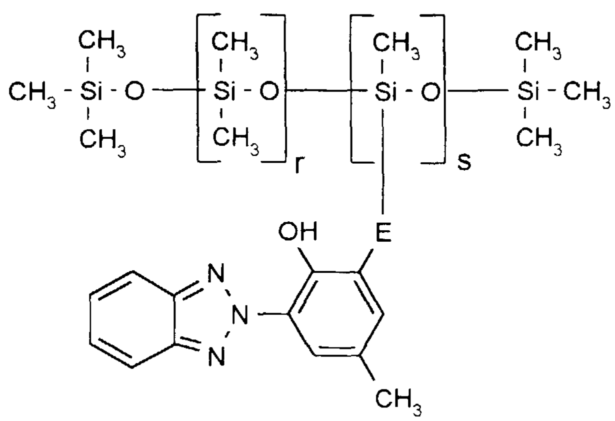 (5)

avec

$$0 \leq r \leq 10,$$

$$1 \leq s \leq 10,$$

et où E représente le radical divalent :

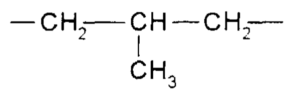

**5.** Composition selon la revendication 4, caractérisée par le fait que le dérivé silicié à fonction benzotriazole répond à la formule suivante :

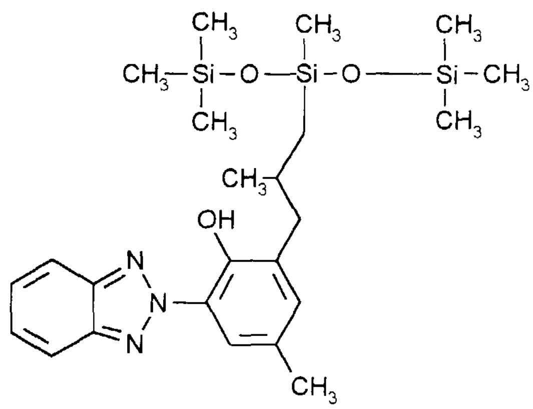

**6.** Composition selon l'une quelconque des revendications 1 à 5, caractérisée par le fait que les dérivés siliciés à fonction benzotriazole sont présents à une teneur allant de 0,1% à 20 %, de préférence de 0,2 % à 15 % en poids, par rapport au poids total de la composition.

**7.** Composition selon l'une quelconque des revendications 1 à 5, caractérisée par le fait que les triesters du triacide benzoïque sont choisis parmi ceux répondant à la formule générale (I) suivante :

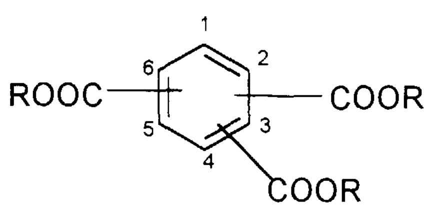

(I)

dans laquelle R désigne un alkyle linéaire ou ramifié, saturé ou insaturé, ayant de 3 à 30 atomes de carbone et de préférence de 8 à 18 atomes de carbone.

**8.** Composition selon la revendication 7, où les triesters du triacide benzoïque sont choisis parmi les triesters de l'acide trimellitique répondant à la formule générale (II) suivante :

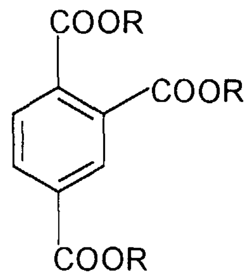

(II)

dans laquelle R a la même signification indiquée dans la formule (I).

**9.** Composition selon la revendication 8, où le composé de formule (II) est choisi parmi :

- le trimellitate de 2-éthylhexyle;
- le trimellitate de décyle ;
- le trimellitate de triisodécyle ;
- le trimellitate de butyl-2 hexyle ;
- le trimellitate de tridécyle ; ou leurs mélanges.

**10.** Composition selon l'une quelconque des revendications 1 à 9, caractérisée par le fait que le triester de triacide benzoïque est présent à des teneurs allant de 0,1 à 99% en poids par rapport au poids total de ladite composition, et de préférence à des teneurs allant de 0,5 à 50 % en poids.

**11.** Composition selon l'une quelconque des revendications 1 à 10, selon laquelle les concentrations en dérivé silicié à fonction benzotriazole et en composé triester du triacide benzoïque, sont choisies de manière telle que l'indice de protection solaire de ladite composition soit de préférence d'au moins 2.

**12.** Composition selon l'une quelconque des revendications 1 à 11, caractérisée par le fait qu'elle présente sous la forme d'une composition solide, pâteuse ou liquide, anhydre ou en émulsion.

**13.** Composition selon l'une quelconque des revendications 1 à 12, caractérisée par le fait qu'elle présente sous forme d'une huile ; d'un gel ; d'une suspension ; d'un produit bi-phasé, d'une dispersion huile-dans-eau ou eau-dans-huile ; d'une émulsion simple ou complexe ; d'une dispersion vésiculaire ; d'un solide coulé ou moulé ; d'une poudre ; d'une mousse ou d'un spray.

**14.** Composition selon l'une quelconque des revendications 1 à 13, caractérisée par le fait qu'elle est un produit de maquillage et/ou de soin de la peau et/ou des lèvres et/ou des phanères.

**15.** Composition selon la revendication 14, sous forme de produit à lèvres.

**16.** Composition selon l'une quelconque des revendications 1 à 15, caractérisée par le fait qu'elle contient en outre des adjuvants cosmétiques choisis parmi les corps gras, les solvants organiques, les épaississants ioniques ou non ioniques, les adoucissants, les antioxydants et notamment les antioxydants ARL, les opacifiants, les stabilisants, les émollients, les silicones, les composés fluorés, les α-hydroxyacides, les agents anti-mousse, les agents hydratants, les vitamines, les parfums, les conservateurs, les tensioactifs, les pigments, les nacres, les charges, les séquestrants, les polymères, les propulseurs, les agents alcalinisants ou acidifiants, les colorants.

**17.** Composition selon l'une quelconque des revendications 1 à 16, caractérisée par le fait qu'elle est un produit antisolaire pour la photoprotection de la peau, des lèvres et /ou des phanères.

**18.** Composition selon l'une quelconque des revendications 1 à 17, caractérisée par le fait qu'elles comprend en outre un ou plusieurs filtres organiques complémentaires actifs dans l'UV-A et/ou UV-B, hydrophiles ou lipophiles.

**19.** Composition selon la revendication 18, caractérisée par le fait que lesdits filtres organiques complémentaires sont choisis parmi les dérivés du camphre, les dérivés de triazine, les dérivés de l'acide cinnamique, les dérivés de l'acide salicylique, les dérivés de la benzophénone, les dérivés du dibenzoylméthane, les dérivés de β,β-diphénylacrylate, les dérivés de l'acide p-aminobenzoïque, les polymères filtres et les silicones filtres autres que les dérivés siliciés à fonction benzotriazole.

**20.** Composition selon l'une quelconque des revendications 1 à 19, caractérisée par le fait qu'elles comprend en outre, à titre d'agents photoprotecteurs complémentaires pigments ou des nanopigments d'oxydes métalliques, enrobés ou non. capables de bloquer physiquement, par diffusion et/ou réflection, le rayonnement UV.

**21.** Composition selon la revendication 20, caractérisée par le fait que lesdits pigments ou nanopigments sont choisis parmi les oxydes de titane, de zinc, de fer, de zirconium, de cérium et leurs mélanges, enrobés ou non.

**22.** Composition selon l'une quelconque des revendications 1 à 21, caractérisée par le fait qu'elle comprend en outre au moins un agent de bronzage et/ou de brunissage artificiel de la peau.

**23.** Composition selon l'une quelconque des revendications 1 à 19, caractérisée par le fait qu'elle est un produit capillaire.

**25.** Utilisation d'une composition telle que définie dans les revendications 1 à 24 pour la fabrication d'une composition cosmétique destinée à la protection de la peau et/ou des lèvres et/ou des phanères contre les radiations UV.

**25.** Utilisation d'au moins un triester du triacide benzoïque tel que défini dans les revendications précédentes dans la fabrication d'une composition cosmétique pour la protection de la peau et/ou des lèvres et/ou des phanères contre les radiations UV contenant un système filtrant les radiations UV lipophile constitué d'au moins un dérivé silicié à fonction benzotriazole, pour améliorer le pouvoir photoprotecteur de ladite composition.

Office européen des brevets

# RAPPORT DE RECHERCHE EUROPEENNE

Numéro de la demande
EP 99 40 1882

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A,D | EP 0 711 779 A (L'OREAL)<br>15 mai 1996 (1996-05-15)<br>* le document en entier * | 1-25 | A61K7/42 |
| A | WO 94 06404 A (L'OREAL)<br>31 mars 1994 (1994-03-31)<br>* le document en entier * | 1-25 | |
| D | & EP 0 660 701 B (L'OREAL) | | |
| A,D | US 4 940 574 A (KAPLAN)<br>10 juillet 1990 (1990-07-10)<br>* le document en entier * | 1-25 | |

DOMAINES TECHNIQUES RECHERCHES (Int.Cl.7)

A61K

Le présent rapport a été établi pour toutes les revendications

| Lieu de la recherche | Date d'achèvement de la recherche | Examinateur |
|---|---|---|
| LA HAYE | 15 décembre 1999 | Fischer, J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 99 40 1882

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

15-12-1999

| Document brevet cité au rapport de recherche | Date de publication | Membre(s) de la famille de brevet(s) | Date de publication |
|---|---|---|---|
| EP 711779 A | 15-05-1996 | FR 2726562 A | 10-05-1996 |
| | | AT 162792 T | 15-02-1998 |
| | | AU 679391 B | 26-06-1997 |
| | | AU 3423695 A | 16-05-1996 |
| | | BR 9504902 A | 02-09-1997 |
| | | CA 2162330 A,C | 09-05-1996 |
| | | CN 1135487 A | 13-11-1996 |
| | | DE 69501541 D | 05-03-1998 |
| | | DE 69501541 T | 14-05-1998 |
| | | ES 2117368 T | 01-08-1998 |
| | | HU 74042 A | 28-10-1996 |
| | | JP 2885672 B | 26-04-1999 |
| | | JP 8208668 A | 13-08-1996 |
| | | PL 311263 A | 13-05-1996 |
| | | US 5610257 A | 11-03-1997 |
| | | US 5714134 A | 03-02-1998 |
| | | ZA 9508886 A | 09-05-1996 |
| WO 9406404 A | 31-03-1994 | FR 2695560 A | 18-03-1994 |
| | | AT 154233 T | 15-06-1997 |
| | | AU 678382 B | 29-05-1997 |
| | | AU 4822493 A | 12-04-1994 |
| | | CA 2144988 A | 31-03-1994 |
| | | DE 69311561 D | 17-07-1997 |
| | | DE 69311561 T | 02-10-1997 |
| | | DK 660701 T | 19-01-1998 |
| | | EP 0660701 A | 05-07-1995 |
| | | ES 2102678 T | 01-08-1997 |
| | | GR 3024469 T | 28-11-1997 |
| | | JP 8504184 T | 07-05-1996 |
| | | US 5618520 A | 08-04-1997 |
| US 4940574 A | 10-07-1990 | CA 2006168 A,C | 22-06-1990 |

EPO FORM P0460

Pour tout renseignement concernant cette annexe : voir Journal Officiel de l'Office européen des brevets, No.12/82